Europäisches Patentamt

European Patent Office

Office européen des brevets

(11) Publication number: **0 643 075 A1**

# EUROPEAN PATENT APPLICATION
## published in accordance with Art. 158(3) EPC

㉑ Application number: **94907668.1**

㉒ Date of filing: **21.02.94**

㊆ International application number:
**PCT/JP94/00284**

㊇ International publication number:
**WO 94/19371 (01.09.94 94/20)**

㊄ Int. Cl.⁶: **C07K 7/10, C12N 15/15, C12N 1/21, C12P 21/02**

㉚ Priority: **22.02.93 JP 31758/93**

㊸ Date of publication of application:
**15.03.95 Bulletin 95/11**

㊤ Designated Contracting States:
**BE CH DE FR GB IT LI**

㉛ Applicant: **NISSIN SHOKUHIN KABUSHIKI KAISHA**
**1-1, Nishi-Nakajima 4-chome,**
**Yodogawa-ku**
**Osaka-shi,**
**Osaka 532 (JP)**

㉜ Inventor: **NAKANO, Shigeru**
**563-2,**
**Ishibe,**
**Ishibe-cho**
**Koka-gun,**
**Shiga 520-31 (JP)**
Inventor: **MABUCHI, Toshiyuki**
**14-10,**
**Kusatsu 3-chome**
**Kusatsu-shi,**
**Shiga 525 (JP)**
Inventor: **TADA, Miki**
**12-23,**
**Ichiriyama 2-chome**
**Ohtsu-shi,**
**Shiga 520-21 (JP)**

Inventor: **TAODA, Yasuo**
**410-1,**
**Kitanakakoji,**
**Ritto-cho**
**Kurita-gun,**
**Shiga 520-30 (JP)**
Inventor: **SUGINO, Dan**
**2-1,**
**Karasaki 4-chome**
**Ohtsu-shi,**
**Shiga 520-01 (JP)**
Inventor: **KONO, Yoshio**
**4-97,**
**Higashino-Kitainouecho,**
**Yamashina-ku**
**Kyoto-shi,**
**Kyoto 607 (JP)**
Inventor: **NISHIMURA, Kaoru**
**470,**
**Nojicho**
**Kusatsu-shi,**
**Shiga 525 (JP)**
Inventor: **OKUSHIMA, Minoru**
**8-9,**
**Hirakata-Motomachi**
**Hirakata-shi,**
**Osaka 573 (JP)**

㊆ Representative: **Rackham, Anthony Charles**
**Lloyd Wise, Tregear & Co.**
**Norman House**
**105-109 Strand**
**London WC2R 0AE (GB)**

EP 0 643 075 A1

**(54) NOVEL PEPTIDE HAVING ELASTASE INHIBITOR ACTIVITY AND PROCESS FOR PRODUCING THE SAME.**

(57) A novel peptide having an elastase inhibitor activity and being useful as a remedy or preventive for diseases caused by elastase is produced by partially modifying the constitutive amino acid of HI-8 present on the C-terminal side of a human urine trypsin inhibitor and having a trypsin inhibitor activity. The invention also provides a gene coding for the peptide, a plasmid containing the gene, a recombinant microbe holding the plasmid, and a process for producing the peptide by using the recombinant microbe.

Fig.2

(Technical Field)

The present invention relates to a novel peptide having a pharmacologically useful activity prepared by altering a part of amino acids which constitute the peptide having a normal protease inhibitory activity, and a producing method thereof.

(Background Art)

Elastase, which is one of the proteolytic enzymes, have been said that it may play an important role in metabolism on the living tissue. In particular, elastase, which is secreted from neutrophil being one of the lymphocytes, have been known that large amount thereof is involved ininfectious disease and inflammation of tissue, and it is closely anticipated in protection against infection and regeneration of damaged tissue. On the other hand, protein having elastase inhibitory activity is also produced in the living body, and there is a mechanism to control not to excessivly degrade the living tissue by neutralizing the excess elastase activity [Ann. Rev. Med. 36, pp. 207-216 (1985)].

As stated above, usually, although homeostasis between an activity of elastase and that of elastase inhibitory protein, which is strictly regulated in the living body, is maintained, when this balance is once lost with some factors, the various disease aspects will be generated. For example, when the elastase activity is enhanced, diseases such as pulmonary emphysema, idiopathic pulmonary fibroma, adults respiration depression syndrome (ARDS) are caused in the lung [Metabolism, 29, pp. 41-49 (1992)], and an increase of neutrophil elastase activity in the joints is thought to be closely anticipated in generation of diseases such as rheumatoid arthritis, deformative arthritis [Agents Actions. 8, pp. 11-18 (1978): Bulletin of Pharmacological Society of Japan, 99, pp. 93-107 (1992)]. Further, elastase is thought to be concerned with generation of acute and chronical inflammatory diseases [Ann. Rev. Med. 36, pp. 207- 216 (1985)].

α 1-anti-trypsin (hereinafter referred to as " α 1-AT") have been well known as one of the elastase inhibitory proteins which modulate an activity of elastase in the living body. α 1-AT is existed in the blood in a large amount, and play a role to inhibit and neutralize the elastase activity accelerated partially [Nature, 298, pp. 329-334(1982)]. However, since α 1-AT is very susceptible to an oxidization, when the air containing the much peroxide owing to smoking and air pollution was inhaled continuously, α 1-AT is oxidized with the inhaled peroxide in the lung, and elastase inhibitory activity thereof may be lost [Am. Rev. Respir. Dis. , 116, pp. 65-72 (1977)].

Further, α 1-AT is oxidized with an active oxygen released from leukocytes swarmed to the portion in the lung inflamed by the inhalation of the polluted air containing inflammable materials, and it losts an elastase inhibitory activity [J. Clin. Invest., 66, pp. 987-995 (1980)]. Such inactivation of α 1-AT leads to a condition of an excess elastase activity in the limited portion in the lung, then the alveolus tissue is degraded by the excess elastase, and raise the lung disease such as pulmonary emphysema [Area of Chmeical Therapy, 5, pp. 1455-1459 (1989)]. When bacteria infect to the lung, and inflammation is therefore caused at the limited portion in the lung, α 1-AT is also inactivated and the lung tissue may be destroyed, because leukocytes such as neutrophil swarme to the inflammatory site by the infection and they secrete a large amount of active oxygen and elastase [Metabolism, 29, pp. 41-49 (1992)].

In order to prevent and cure the diseases by an elastase aforementioned, it have been said that administration of an elastase inhibitory substance such as α 1-AT into blood or portion changed pathologically may be effective [Can. Med Assoc. J., 146, pp. 841-844 (1992)]. However, it is necessary to use the α 1-AT derived from human to avoid an antigen-antibody reaction to be caused as a side-effect, therefore, to obtain enough α 1-AT for prevention and cure had been difficult.

Then, since pathogenic virus derived from human may remain in the purified α 1-AT products, when an elastase inhibitory substance such as α 1-AT is extracted and purified from human blood, this is a factor that has been difficult to use α 1-AT derived from human for prevention and cure of diseases induced by elastase.

Further, although spray-inhalation method by nebulizer is used commonly for administrating the drugs against lung diseases, since α 1-AT is very susceptible to oxidization as stated above, elastase inhibitory activity is inactivated by oxidation with the spray, and, if an administration is performed through the other route, is also inactivated with an active oxygen generated from various cells in the living body.

On the other hand, application of chemically synthesized low molecular weight elastase inhibitors to the above referenced diseases are also examined. However, since substances to be administrated in these methods are foreign subtances to the living body, when these are administrated into the living body, undesirable side-effect, such as toxicity, may be raised. Then, these chemically synthesized low molecular weight elastase inhibitors may also inhibit an activity of the other proteolytic enzymes having serious

3

physiological activities besides the elastase, and this less-specificity restricts an usage of these chemically synthesized low molecular weight elastase inhibitors as the drugs against the disases induced by elastase.

Though elastase inhibitor is thought to be quite useful for preventing and curing the disease induced by elastase, such as pulmonary emphysema, there are various problems above for the pharmaceutical application of the inhibitor derived from human such as $\alpha$ 1-AT and chemically synthesized low molecular weight elastase inhibitors, accordingly, clinical application thereof as a medicine have been remainded to be accomplished.

(Disclosure of Invention)

The present invention was established in view of the problems in the art aforementioned, and purposes thereof are to provide a novel peptide which is quite useful pharmacologically as a drug for the prevention and the cure of diseases caused by elastase, and have strong inhibitory activity against the human neutrophil elastase and anti-oxidation, and less ability to raise an antigen-antibody reaction, prepared by altering a part of amino acid sequence of C-terminal domain (hereinafter referred to as "HI-8") in human-urinary-trypsin-inhibitor (Ulinastatin, hereinafter referred to as "UTI") distributed in the market as a medicine and confirmed desirable effects against the human; and a producing method thereof through the genetic engineering technique using recombinant microorganisms.

More specifically, the present invention relates to a novel peptide, a gene encoding said peptide, a recombinant microorganism containing said gene, and a producing method of said novel peptide using said recombinant microorganism, wherein said peptide have a pharmacologically useful elastase inhibitory activity obtained by altering, with a genetic or a proteinic engineering technique, a part of amino acid sequences within approximately 7.3kDa fragment [Hoppe-Seyler's Z. Physiol. Chem. Bd. 362, S. pp. 1351-1355 (1981)], existed in C-terminal domain of human-urinary-trypsin inhibitor prepared by limited degradation of UTI under the presence of excess proteolytic activity in vivo or trypsin in vitro, and have a trypsin inhibitory activity.

UTI is a trypsin inhibitor to be contained in the human urine, and is a protein which have been applied as a purified products from the human urine to the medicine usage for acute circulatory defects as well as pancreatitis, and desirable effect on intravenous administration have also been confirmed [Digest. Dis. Sci., pp. 26-32 (1984): Jpn. J. Pharmacol., 39, pp. 137-144 (1985)]. Approximately 7.3 kDa domain of C-terminal portion of UTI having trypsin inhibitory activity is contained in the human urine together with UTI, and is thought to be produced through a limited hydrolysis by a trypsin-like enzyme in the living body.

The present inventors focused on HI-8 to be thought as a very safety for the human, and tried to reduce trypsin inhibitory activity in comparison with that of normal HI-8, and to generate independent novel strong elastase inhibitory activity by altering a part of amino acid sequence which is adjacent to a core portion relating to the activity. First of all, three-dimentional structure of a protein complex of HI-8 and human neutrophil elastase is deduced with a computer program for deducing three-dimentional structure of proteins, then the site which is going to be specifically bound to human neutrophil elastase, and kinds of amino acids, which are non-sensitive to the oxidation, for alteration at this site were assumed based on study concerning the binding site therebetween in spatial axis.

Furthermore, in order to perform the production by a genetic recombinant technique which is suitable for a large scale production, simple purification, and avoid an incorporation of the virus derived from the human, using Escherichia coli as a recombinant host, DNA which encode amino acid sequence of HI-8 and appropriate secretive signal peptide were chemically synthesized with codon usage to be suited thereto, and said DNA was cloning into an appropriate expression vector. Then, site-directed mutagenesis were performed against the plasmid containing this synthesized DNA to correspond to amino acid sequences assumed from said computer analysis. Peptides encoded by mutated DNA so obtained were expressed by recombinant host bacteria, evaluated an inhibitory activities of each purified altered-peptide against the human neutrophil elastase, and selected novel peptide having strong elastase inhibitory activity.

As a result, Ki value against the human neutrophil elastase which is about $6.9 \times 10^{-7}$ M on non-altered HI-8 was able to reduce to about $6 \times 10^{-10}$ M by altering 3rd, 11th, 15th, 18th, and 46th amino acid residues from N-terminal of this peptide to amino acids respectively shown in the Example.

In the other words, the present inventors have discovered that strong elastase inhibitory activity could be obtained by intentionally altering a part of amino acid sequences of HI-8. Amino acid sequence, which have been elucidated in the present invention, of altered HI-8 having elastase inhibitory activity is set out in SEQ:ID No. 1.

Apparently from the following Examples concerning an inhibitory effects of the altered peptides on the human neutrophil elastase of the altered peptides preferable 15th amino acid in the sequence of SEQ:ID

4

No. 1 is hydrophobic amino acid residue, such as isoleucine, leucine, valine, and the strongest inhibition against the human neutrophil elastase was obtained by an altered peptide, wherein 11th amino acid was changed with glutamic acid, and 46th amino acid was changed with glutamine.

Although this altered peptide of HI-8 comprises amino acid sequence having sixty-six amino acids set out in SEQ:ID No. 1, an inhibitory activity against the human neutrophil elastase will be expressed, even if a part of amino acid sequence in N-terminal side or C-terminal side is deleted. In the other words, the present invention encompasses, as a matter of course, a core structure of altered HI-8 which inhibits the human neutrophil elastase.

Further, purposes of the present invention will be achieved by substituting, with a corresponding portion in the other serine protease inhibitor amino acid sequences which lie adjacent to an active site of altered HI-8, for example, a peptide containing both of sequences of four amino acids extended to N-terminal and C-terminal from 15th amino acid in the peptide of the present invention.

Altered HI-8 peptide of the present invention will be produced by chemically synthesizing DNA which encodes the sequences thereof, inserting it into downstream of an appropriate promoter, introducing them a preferable host such as Escherichia coli, Bacillus subtilis, and animal cell, and culturing this transformants.

Then, DNA which codes a target altered peptide can be obtained by isolating DNA for UTI from an appropriate human cDNA library, and introducing required alteration into this DNA with site-directed mutagenesis technique.

To secrete the altered peptide of the present invention into the culturing liquid by recombinant host is useful for effectively purifying the altered peptide. At that time, an appropriate signal sequence for the secretion is inserted into upstream of the DNA encoding the altered peptide. For example, secretion of the peptide into the culturing liquid is possible if a host is animal cell, in addition thereto, secretion of the peptide into the culturing liquid or periplasmic space is also possible when a host is Escherichia coli.

To produce the altered peptide of the present invention as a stable fused protein with the host by connecting it to the other appropriate protein is also useful. Expressed fused protein can be isolated and purified through a technique using the character such as antigenecity of the connected protein, and targeted altered peptide can be isolated and purified by site-directly cleaving the fused protein so obtained with enzyme which is appropriate to proteolysis or chemically cleaving technique which is specific to the sequence of the peptide.

Further, the altered peptide of the present invention can be produced, by adding SD sequence as well as an appropriate promoter such as lac, trc, tac to upstream of DNA sequence which codes this altered peptide, furthermore adding an appropriate termination signal to downstream thereof, introducing it into host microorganism as the duplicatable plasmid corresponding to the host which is ligated thereto, and culturing the obtained transformants. When the targeted peptide is expressed in the bacterial cells, ATG, initiation signal for translation, is added to 5' end of the gene. When the altered peptide is secreted to the culturing liquid or periplasm of the host cell, DNA which codes signal peptide is adjoined to the upstream of the gene. By altering the amino acid sequence of this connected portion with site-directed mutagenesis method, digestion of the signal peptide to give a desired N-terminal sequence of the altered peptide may also be possible. Then, by an addition of inducer to the medium to enhance the function of the promoter and an operation to increase an amount per unit time on the expression, the altered peptide in a form of inclusion body can be produced in cytoplasm under absence of signal peptide or periplasm on the presence of the signal peptide.

When the altered peptide of the present invention is produced by using a microorganism host, an express ion product wherein preferable S-S bond between the cystein residues is not formed, and, therefore, is not formed three-dimentional structure as same as the native type may be produced. An expression in a manner as the inclusion body above is advantageous to partial purification, however, to obtain an active altered peptide is difficult in view of the similar reasons. In these cases, by purifying it under the reducing condition to reduce the S-S bond with an appropriate reducing agent, and by applying a re-oxidizing method to the peptide according to the characteristic of it, the activity can be recovered.

The altered peptide according to the present invention is easily separated and purified from the reaction solution, bacterial cells, or culturing liquid with a combination of the conventional separation and purification techniques. These techniques to be employed comprise a method of using solubility of the product such as salting-out. solvent-precipitation, a method of utilizing a difference on molecular weight such as dialysis, ultrafiltration, gel-filtration or SDS-polyacrylamide gel electrophoresis, a method of utilizing an electric affinity such as ion-exchange chromatography, a method of utilizing a difference on hydrophobicity such as reverse phase chromatography, or a method of utilizing a difference on isoelectric point such as isoelectrofocusing.

(Brief Description of the Drawings)

Fig. 1 illustrates section on ten oligonucleotides in a gene designed to have structure set out in SEQ:ID No. 2.

Fig. 2 illustrates a process for constructing the expression plasmid of the present invention.

(Best Mode to Practice the Invention)

Example 1: Construction of Gene encoding C-terminal peptide (HI-8) of UTI

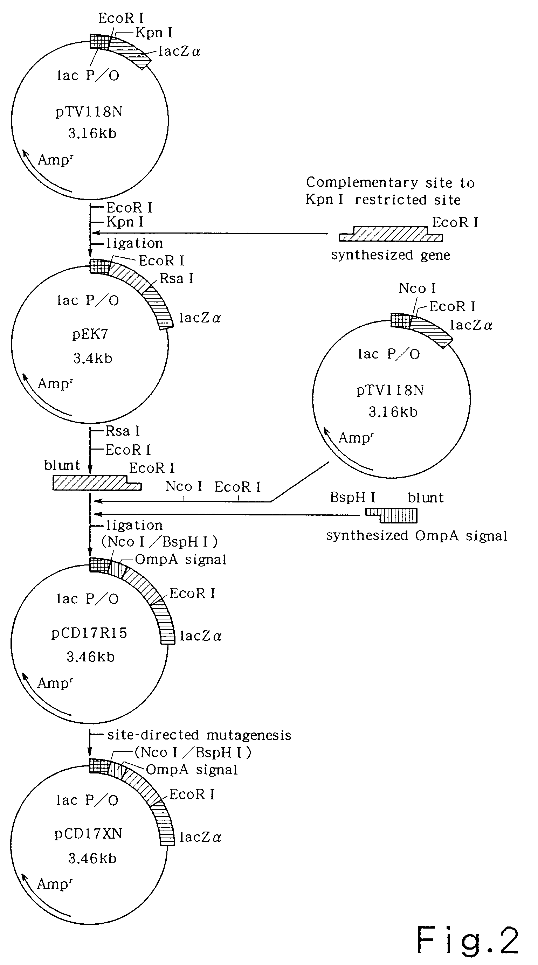

Firstly, construction of the gene coding a peptide of C-terminal side of UTI, as a starting gene for preparing mutants, is explained with reference to Fig. 2.

Based on the amino acid sequence of UTI disclosed in the thesis of Hoppe-Seyler's Z. Physiol. Chem., 362, pp. 1351-1355 (1981), a gene having a structure set out in SEQ:ID No. 2 which codes C-terminal side region (Thr$^{78}$-Leu$^{143}$) containing trypsin inhibitory active site of UTI was designed, with selecting codon which is used frequently in Escherichia coli, successively arranging one of the termination signal codon for translation, TGA, at 3' end of the structural gene, and adding Trp terminator to the downstream thereof, and providing restriction-enzyme-recognition-sites (KpnI digestable site at 5' end, EcoRI digestable site at 3' end) at both ends for the construction of said starting gene.

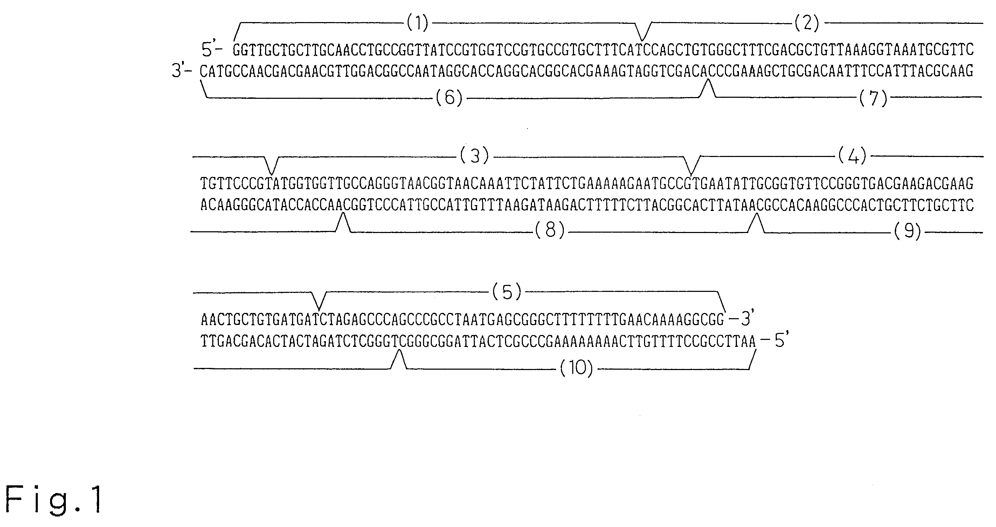

Then, this gene was divided according to sections shown in Fig. 1, and chemically synthesized each oligonucleotide (total 10 kinds) having one of the base sequences set out in SEQ:ID Nos. 3 through 12 [corresponding to the sequences (1) through (10) in Fig. 1]. These oligonucleotides were synthesized by a method of using phosphoamidite with automatic DNA synthesizer (Model 381A, Applied Biosystems). Purification of the synthesized oligonucleotide was performed according to a manual for purification of Applied Biosystems. That is to say, protective groups of the bases were released by heating concentrated ammonia solution containing synthesized oligonucleotides at 55°C for a whole day and night, and prepurification was performed with OPC cartridge (Applied Biosystems). Further, if necessary, 5' end of the synthesized oligonucleotide was phosphorylated through a reaction at 37 °C for one hour in a solution of 1mM MgCl$_2$, 0.5mM dithiothreitol, 1mM ATP and 50mM Tris-HCl (pH 7.6) containing 16 units of polynucleotidekinase (Toyobo).

Then, it was applied to polyacrylamidegel electrophoresis (gel phase 20%) containing 7M urea stained the gel with ethidium bromide, excised a band portion corresponding to targeted oligonucleotide appeared on a long wave length (365nm) ultra violet generator, and well fined it. 1ml of the solution for the extraction of DNA [20mM Tris-HCl (pH 8.0), 1.5mM EDTA] was added to these fined samples, then, these were shaked overnight at 37 °C, and the solution containing the synthesized oligonucleotides were obtained through a purification by applying the supernatant on centrifugation to desalting-column.

Each pair of complementary strands shown in Fig. 1, for example, base sequences (1) and (6), are mixed in 50mM Tris-HCl (pH 7.6), 10mM MgCl$_2$ to become equivalent mole each other, treated the solution at 90°C for 5 minutes, and annealed by gradually cooling it to room temperature. Annealed synthesized DNA fragments were isolated on poly-acrylamide gel electrophoresis (gel phase 10%) not containing urea, and purified according to the similar method referred previously in purification of synthesized oligonucleotides.

Plasmid pTV118N (Takara-Shuzo) was digested with restriction enzyme EcoRI and KpnI, separated linearized fragment on agarose gel electrophoresis, and excised the gel containing the target DNA band. Samples prepared by freezing the gel block at -80 °C for one hour and rapidly heated up to 37 °C was filtrated with centrifugation filter (Milipore) of 0.1 μm pore size.

Filtrated samples were extracted with phenol reagent, then precipitated with ethanol, and the linearized plasmid was purified and collected. Collected plasmids and five pairs of the synthesized DNA fragments [i.e., pairs of base sequences (1) and (6), (2) and (7), (3) and (8), (4) and (9), (5) and (10), respectively shown in Fig. 1] were mixed in a solution containing 50mM Tris-HCl (pH 7.6). 10mM MgCl$_2$, 10mM dithiothreitol, 1mM ATP, and reacted overnight at 4 °C for ligation with 10 units of T4 DNA ligase (Takara-Shuzo).

10 μl of reacted solution was added to 100 μl of Escherichia coli (JM109) competent cell suspension (Takara-Shuzo), and the transducion was performed according to a manual of Takara-Shuzo. Some of the transformants grown in selective medium (LB-agar plate) containing ampicillin was cultured, and a plasmid DNA incorporated thereinto was extracted according to the method using alkali [Yodo-sha, Genetic-Engineering Handbook, pp. 19-26 (1991)]. Confirmation as to whether or not the target gene and a plasmid containing it were appropriately constructed was performed by deducing a pattern of agarose gel electrophoresis on sample digested with several restriction enzymes, then directly confirming through DNA sequencing by the method using dideoxy nucleotides [Proc. Nat. Acad. Sci. USA. 74, pp. 5463-5467 (1977)-]. Plasmid constructed and selected in the experiment above was named as pEK7.

Double strand DNA fragments which were annealed the synthesized single strand DNA set out in SEQ. ID:Nos. 13 and 14 were constructed according to the method aforementioned, wherein it coded amino acid sequence of signal peptide on Escherichia coli outer membrane protein A (OmpA) and had the BspHI cohesive end at the 5' end of coding strand and blunt end at 3' end thereof.

This double strand DNA fragment and 0.25kb DNA fragment containing synthesized gene produced by digesting said plasmid pEK7 with restriction enzymes RsaI and EcoRI were ligated to between the sites of restriction enzymes NcoI and EcoRI in pTV118N according to the foregoing method.

This reaction solution was used for a transduction for Escherichia coli (JM109) according to the foregoing method, then, some of colonies grown on LB-agar plate containing ampicillin were cultured, and plasmid DNA incorporated there into were extracted.

Through the analysis for the pattern on agarose gel electrophoresis directed to the samples digested with restriction enzymes and for the result of DNA sequencing by the method using dideoxy nucleotides, plasmid, which was confirmed that it have the desirable structure, was named as pCD17R15.

Example 2: Preparation of Anti-Sera against Partial Synthesized Peptide ($Ser^{124}$-$Glu^{141}$) of UTI

For detection and identification of the expressed peptide, anti-sera against partial peptide ($Ser^{124}$-$Glu^{141}$ of UTI) of the expressed peptide was prepared.

Firstly, based on the primary structure of UTI, a region which was C-terminal side beyond $Thr^{78}$ and had high hydrophilic residues, in the other words, a region of $Ser^{124}$-$Glu^{141}$ containing amino acid sequence having eighteen residues set out in SEQ ID: No. 15 was selected. Synthesis of the peptide having this amino acid sequence was performed through the synthesizing method on solid phase using an automatic peptide synthesizer (Model431A; Applied Biosystems). Synthesized peptide was separated from the support according to a manual of Applied Biosystems, and purification of the separated synthesized peptide was performed with a reverse phase liquid chromatography.

10mg of purified synthesized peptide and 3 mg of ovoalbumin (Sigma, Type III) were dissolved in 1ml of distilled water, added thereto 30mg of 1-ethyl-3-(3-dimethylaminopropyl)-carbodiimide hydrochloride (Nakaraitesk), reacted overnight at room temperature under shading, and prepared the products wherein synthesized peptide was conjugated to ovoalbumin.

After dialysing with distilled water, emulsion prepared by adding equivalent amount of Freund Complete Adjuvant (Difco) to a solution of this synthesized peptide-ovoalbumin conjugated products was used for first administration (1ml), then emulsion prepared by adding Freund Incomplete Adjuvant (Difco) was used for second and succeive administrations (0.5ml). These emulsion were administrated hypodermically every one week to Japanese white rabbit (11 weeks old, female), and collected blood when the titer of the specific antibody was raised. Collected blood was left for one hour at room temperature, then overnight at 4°C, and removed corpuscle mass by centrifugation, and used the supernatant thereof as anti-sera in succesive experiments. This anti-sera reacted little or nothing with protein originated from Escherichia coli, and had strong reactivity with said synthesized peptide and HI-8.

Example 3: Expression of Recombinant HI-8

Escherichia coli (JM109) having pCD17R15 was inoculated into 20 ml of TB medium [1.2% Bactotryptone (Difco), 2.4% yeast extract (Difco), 2.31g/l $KH_2PO_4$, 12.54g/l $K_2HPO_4$, 0.4% glycerol. (pH 7.0)] containing 100 $\mu$g/ml ampicillin, and precultured overnight at 37°C. To become $OD_{600nm}$ to 0.1, an appropriate amount of this preculture was added to 400ml of TB medium for next culture, and aerobically cultured at 37°C. When $OD_{600nm}$ of this culture became 0.5, isopropylthiogalactoside (IPTG: Nakaraitesk) was added to the medium to be 100 $\mu$M of final concentration, and the culture was continued to the late logarithmic growth phase. After the culture, bacterial cells were harvested by centrifugation, and washed with buffer containing 50mM Tris-HCl (pH 8.0), 1mM EDTA, 50mM NaCl.

Washed cells were resuspended in said buffer, volume of which is 1/20 of the original culturing liquid, and the bacterial cells were fined under an iced condition by sonication generator. This suspension of the bacterial debris were centrifuged, and recombinant HI-8 was obtained as a precipitate. Then, this precipitate was dissolved into solution of 20mM Tris-HCl (pH 8.5) containing 8M urea, and prepared crude recombinant HI-8 solution.

The presence of target recombinant HI-8 in this solution was confirmed by Western blot technique using anti-sera referred in Example 2. More specifically, the crude solution was subjected to SDS-polyacrylamide gel electrophoresis (gel phase 20%) [Nature, 227, p. 680, (1970)], after the electrophoresis, and separated protein bands appeared in the gel were transferred to the polyvinylidendifluoride membrane (Milipore: hereinafter referred to as "PVDF membrane") with electro blotting device (Tefco) for protein. PVDF membrane was washed with water, and incubated it for one hour in solution prepared by adding skim milk (Difco) to become 1% into 50mM Tris-HCl (pH 7.6) buffer containing 150mM NaCl (hereinafter referred to as "TBS buffer"). Then, the membrane was transferred in TBS buffer containing 1% skim milk and said anti-sera, incubated for two hours at 4 °C, and the PVDF membrane was excessively washed with TBS buffer containing 0.05% Tween 20. Further, it was incubated for two hours at 4 °C in TBS buffer containing alkaline-phosphatase-labelled-goat-anti-rabbit IgG solution (Bio-Rad) and 1% skim milk. After excessively washing the PVDF membrane again with TBS buffer containing 0.05% Tween 20, this was immersed into reaction solution of alkaline phosphatase [Tris-HCl (pH 9.5) solution containing 5ml of 10mM NaCl, 0.5mM $MgCl_2$ wherein 20 $\mu$l of nitrotetrazolium blue solution (prepared by dissolving nitrotetrazolium blue (Dojin) to 50mg/ml in 70% dimethylformamide solution) and 20 $\mu$l of 5-bromo-4-chloro-3-indolyl-phosphate solution (prepared by dissolving 5-bromo-4-chloro-3-indolyl-phosphate (Sigma) to 50 mg/ml in dimethylformamide) were added] and initiated a colorimetric reaction. As a result thereof, band which reacts with said anti-sera was appeared at a position of approximately 8 kDa. This molecular weight was substantially identical to the molecular weight calculated from amino acid sequence of recombinant HI-8.

Recombinant HI-8 expressed by using this pCD17R15 was named as R15.

Example 4: Uniformalization of N-Terminal Amino Acid in Expressed Peptide

Crude solution of recombinant HI-8 referred in Example 3 was subjected as a sample to SDS-polyacrylamidegel electrophoresis substantially according to a method of Example 3, and expressed peptides were transferred to PVDF membrane with an electroblotting. Excised PVDF membrane containing this expressed peptide was applied to protein sequencer (Model 471A: Applied Biosystems), and analyzed N-terminal amino acid sequence. As a result thereof, in light of proportion on the primary yield, approximately 80% of this sample have N-terminal amino acid sequence of the sequence set out in SEQ. ID: No. 1, and it was indicated that the digestion of signal peptide was completed along with the design, however, the remaining approximately 20% was started at fourth amino acid from N-terminal of the originally designed peptide. This seems to have happened on the grounds that an additional amino acid sequence, which is similar to the digestion-recognition-site of signal peptidase derived from the host, is existed around N-terminal of the target peptide.

Accordingly, in order to uniformarize the digestion site of the signal peptide, alanine residue, which is third residue from N-terminal of the peptide and is thought to lead the misrecognition on the signal peptide, was altered to glycine residue with site-directed mutagenesis using the synthesized oligonucleotides.

A kit of Mutan K ™ (Takara-Shuzo) was employed for this alteration.

More specifically, firstly, according to a manual of Takara-Shuzo, Escherichia coli (CJ236) having pCD17R15 was infected with helper phage M13K07, and obtained single strand pCD17R15. Then, the oligonucleotide, wherein 5' end of synthesized oligonucleotide having the sequence of SEQ. ID: No. 16 was phosphorylated, it was annealed to said single strand pCD17R15 and synthesized complementary strands with DNA polymerase and DNA ligase of the kit. This synthesized DNA was introduced into Escherichia coli (BMH71-18 mutS), cultured at 37 °C for one hour, infected with M13K07, and further continued the culture at the same temperature for 16 through 20 hours. After the culture, supernatant obtained by centrifuging the culturing liquid was appropriately diluted, then added to the culturing liquid of Escherichia coli (MV1184), and 10 minutes later, plated a desirable amount thereof, and formed colonies at 37 °C. Some colonies were selected from the grown colonies, and nucleotide sequences with the mutated site on the plasmid incorporated into the selected bacteia were confirmed with the method used dideoxy nucleotide. Then, plasmid wherein the desirable mutation was introduced therein, was named as pCD17R15G3. N-terminal amino acid sequence of the peptide which was expressed by Escherichia coli (JM109) having pCD17R15G3 was analyzed by the similar method referred previously, and it was demonstrated that third amino acid residue had been altered to glycine from alanine, and that intended signal digestion had been completed, because N-terminal amino acid sequence did not indicate any sequence except for the sequence set out in SEQ:ID No. 1.

Altered R15 peptide expressed by using this plasmid was named as R15G3.

Example 5: Preparation of Peptide Having Altered Amino Acid Residues

Site directed mutagenesis was performed by applying the synthesized oligonucleotide to the single strand pCD17R15G3, and was produced the gene for the peptide having altered amino acid residue. Synthesized oligonucleotides to be mutated have the sequences set out in SEQ. ID: Nos. 17 through 22, then, name and sequence of primer (synthesized oligonucleotide) corresponding to the mutated amino acid residues to be induced are listed in the following Table 1.

TABLE 1

| Primer | Sequence |
|---|---|
| I15 primer | (15)<br>5'-GGTCCGTGC ATT GCTTTCATC-3' |
| L15 primer | (15)<br>5'-GGTCCGTGC CTG GCTTTCATC-3' |
| V15 primer | (15)<br>5'-GGTCCGTGC GTT GCTTTCATC-3' |
| E11 primer | (11)<br>5'-CCGGTTATC GAA GGTCCGTGC-3' |
| Q46 primer | (46)<br>5'-TAACGGTAACAAATTC CAG TCTGAAAAAGAATGCCG-3' |
| F18I15 primer | (15)　　　(18)<br>5'-TGC ATT GCTTTC TTC CAGCTGTGG-3' |

cf: Figure in parenthesis are coprresponding to position of the altered amino acid residues.

The altered peptides were indicated herein by one letter symbol on altered amino acid residue (X) followed by the residue numbers from N-terminal on altered residue sites. In the case of peptide altered at several residues, indication of 15th altered amino acid residue was noted ahead. For example, a peptide wherein third and fifteenth amino acids from N-terminal were altered with glycine and isoleucine respectively is indicated as I15G3, and a peptide wherein eleventh amino acid is further altered with glutamic acid is indicated as I15G3E11.

Alteration was performed with a method using Mutan K kit substantially according to the method referred in Example 4, and the required amino acid residues are altered thereby. Alteration on two or more amino acid residues was accomplished by additionally altering the non-altered residues on the mutated plasmid wherein one of the residues has been altered. Nucleotide sequence on the each altered site in the mutated plasmid so obtained was confirmed by the method using dideoxy nucleotides. Mutated plasmids so obtained were indicated, by kind of introduced amino acid residue (X) and altered position (N: number of residues from N-terminal), as pCD17XN.

The present inventors have been developed ten novel plasmids according to the alteration technique aforementioned and named them as pCD17I15G3, pCD17L15G3, pCD17V15G3, pCD17V15G3E11, pCD17V15G3Q46, pCD17V15G3E11Q46, pCD17I15G3E11, pCD17I15G3Q46, pCD17I15G3E11Q46, and pCD17I15G3F18 respectively.

Further, Escherichia coli which have been introduced there into any of pCD17I15G3, pCD17V15G3Q46, pCD17V15G3E11Q46, pCD17I15G3Q46, or pCD17I15G3E11Q46 were prepared, and these Escherichia coli had been deposited on February 17, 1993 in National Institute of Bioscience and Human-Technology Agency of Industrial Science and Technology, at 1-3, Higashi 1 chome, Tsukuba-shi, Ibaraki-ken, JAPAN. Particulars of which are as follows.

Escherichia coli JM109-pCD17I15G3 containing plasmid pCD17I15G3 (FERM BP-4556) ;

Escherichia coli JM109-pCD17V15G3Q46 containing plasmid pCD17V15G3Q46 (FERM BP-4557) ;
Escherichia coli JM109-pCD17V15G3E11Q46 containing plasmid pCD17V15G3 E11Q46 (FERM BP-4560) ;
Escherichia coli JM109-pCD17I15G3Q46 containing plasmid pCD17I15G3Q46 (FERM BP-4558) ; and
Escherichia coli JM109-pCD17I15G3E11Q46 containing plasmid pCD17I15G3 E11Q46 (FERM BP-4559).

## Example 6: Purification of Recombinant Altered HI-8 from Crude Solution

Crude solution of recombinant altered HI-8 was prepared from Escherichia coli JM109 having any of the recombinant plasmid by using Escherichia coli JM109 having plasmid pCD17XN prepared in the present invention according to a method substantially identical to the method referred in Example 3.

More specifically, this crude solution was dialyzed with 20mM ammonium acetate (pH 5.5), centrifuged, and applied the supernatant thereof to ion-exchange chromatography [elution with linear concentration gradient for 10 minutes from 20mM ammonium acetate (pH 5.5) to ammonium acetate (pH 5.5) containing 0.5M NaCl] using Mono-Q column (Pharmacia). Further, gel-filtration using Superose 12 (Pharmacia) equilibrated with 20mM ammonium acetate (pH 5.5) containing 200mM NaCl was applied to the products so obtained, then recombinant altered HI-8 solution which indicates single band in SDS-polyacrylamide-gel was obtained.

## Example 7: Purification of Expressed Peptide and Recovery on Activity Thereof

2-mercaptoethanol (Wako) was added, to become 1% of final concentration thereof, to each crude solution of the recombinant altered HI-8 prepared according to Example 3, left for one hour at room temperature, applied it to a column (16 × 1000mm) filled with Sephacryl S-100HR (Pharmacia) equilibrated with 20mM Tris-HCl buffer (pH 8.5) containing 200mM NaCl, and gel-filtrated with the eluted buffer so obtained. Then, 2ml of eluted fraction (containing 500 $\mu$g purified peptide), which indicates single band of approximately 8kDa molecular weight on SDS-polyacrylamide gel electrophoresis, was added to 14ml of solution for recovering the activity [solution containing 50mM Tris-HCl (pH 8.5), 1M NaCl, 1mM EDTA, 2mM reduced glutathione, 0.2mM oxidized glutathione], then further added thereto 50 $\mu$g protein disulfide isomerase (Takara-Shuzo), and reacted for 15 through 20 hours at room temperature, and concentrated and desalted the products so obtained by using a membrane for ultrafiltration. According to a necessity, the recombinant altered HI-8 was purified through ion-exchange chromatography and gel-filtration, and these were used as a sample having recovered activity.

## Example 8: Determination of Inhibitory Activity against Human Neutrophil Elastase and Bovine Pancreas Trypsin

Determination of Ki value against human neutrophil elastase was accomplished as follows.

Both 10 $\mu$l of solution of human neutrophil elastase (Attends Research), concentration of which was adjusted to 25 $\mu$M with the following buffer, and 10 $\mu$l of test sample adjusted the concentration thereof appropriately were added to buffer containing 30 $\mu$l of 100mM Tris-HCl (pH 7.5), 500mM NaCl 0.01% BSA, and left for 20 minutes at 30 °C. Then, 200 $\mu$l of MeOSuc-Ala-Ala-Pro-Val-7AMC solution (Cambridge Research Biochemicals) adjusted with said buffer the concentration thereof to 0.2mM, 0.3mM and 0.4mM [containing 1% dimethyl-sulfoxide (hereinafter reffered to as "DMSO")] was added to the mixture, reacted at 30 °C, measured fluorescence at 450nm excited at 365nm during 0 through 3 minutes with spectrophotofluorometer (F-3000, HITACHI), and calculated an initial reaction velocity. Ki value was determined by preparing a graph of every substarte concentration having a vertical axis on reciprocal number of the initial reaction velocity and a horizontal axis on sample concentration, and reading an intercrossed point by three straight lines so obtained.

On the other hand, Ki value against the trypsin was determined as follows.

Firstly, buffer containing 20 $\mu$l of 10mM CaCl$_2$, 50mM Tris-HCl (pH 8.0) was mixed with 10 $\mu$l of test sample (1.2 ~ 6 $\mu$M) and 10 $\mu$l of 4.2 $\mu$M bovine pancreas trypsin solution (prepared by dissolving bovine pancreas trypsin in 1mM HCl; Washington Biochemicals), and incubated at 30 °C for three minutes. 110 $\mu$l of 1M Tris-HCl (pH 8.0) buffer containing 100mM CaCl$_2$ was added to this solution, then added 350 $\mu$l of any of 0.6mM, 1.2mM and 2.3mM Bz-DL-Arg-pNA. HCl (Nakaraitesk) solution (containing 10% DMSO), and reacted at 30 °C. Change on absorbance at 405 nm along with the time track was recorded, and determined therefrom initial reaction velocity of the enzyme. Then, Ki value against trypsin was determined according to a procedure which is similar to the procedure for human neutrophil elastase above.

Ki value, obtained by the above procedures, on α 1-AT, UTI, recombinant HI-8 (R15), recombinant HI-8 (R15G3), and altered HI-8 of the present invention against human neutrophil elastase were listed in the following Table 2.

Ki value on UTI, recombinant HI-8 (R15), recombinant HI-8 (R15 G3) against bovine pancreas trypsin, which are not referred therein, were $9.2 \times 10^{-8}$ M, $3.6 \times 10^{-7}$ M $3.6 \times 10^{-7}$ M respectively, however there were no inhibitory activity against bovine pancreas trypsin by the altered HI-8 of the present invention.

TABLE 2

| Inhibitor (Altered Peptide) | Inhibitory Activity against Human Neutrophil Elastase Ki(M) |
|---|---|
| α 1-AT | $3.1 \times 10^{-9}$ |
| UTI | $1.2 \times 10^{-7}$ |
| R15 | $6.9 \times 10^{-7}$ |
| R15G3 | $6.5 \times 10^{-7}$ |
| I15G3 | $2.1 \times 10^{-9}$ |
| L15G3 | $4.1 \times 10^{-9}$ |
| V15G3 | $3.3 \times 10^{-9}$ |
| V15G3E11 | $1.2 \times 10^{-9}$ |
| V15G3Q46 | $2.5 \times 10^{-9}$ |
| V15G3E11Q46 | $8.0 \times 10^{-10}$ |
| I15G3E11 | $1.8 \times 10^{-9}$ |
| I15G3Q46 | $1.7 \times 10^{-9}$ |
| I15G3E11Q46 | $6.0 \times 10^{-10}$ |
| I15G3F18 | $2.5 \times 10^{-9}$ |

As shown in the result of Table 2 and those on trypsin inhibitory activity aforementioned, in comparison with non-altered recombinant HI-8, there was little inhibitory activity against bovine pancreas trypsin by a recombinant peptide wherein fifteenth arginine in the recombinant HI-8 was altered with isoleucine, leucine or valine, and the inhibitory activity have been remarkably reduced. Further, Ki value against human neutrophil elastase by said altered peptides were reduced from 1/168 through 1/1150 of the activity by recombinant HI-8 (R15), there were remarkable increase in the inhibitory activity thereof. In particular, there have been discovered that, in addition to an alteration of the fifteenth position, by further altering the eleventh position with glutamic acid and the fourty-sixth position with glutamine, Ki value against human neutrophil elastase can be reduced to $6 \sim 8 \times 10^{-10}$ M.

Example 9: Resistance against Oxidizer

5 μl of sample, concentration of which was adjusted appropriately, was mixed with 5 μl of 5 mM chloro-succinate-imide [prepared by dissolving chloro-succinate-imide (Nakaraitesk) in 100mM Tris-HCl (pH 7.5) buffer containing 500mM NaCl], then left for 10 minutes at room temperature, and treated the sample with the oxidizer. Then, 5 μl of human neutrophil elastase concentration of which was adjusted with said buffer, and 35 μl of said buffer were further added thereto, and left it for 15 minutes at 30 °C. 450 μl of MeOSuc-Ala-Ala-Pro-Val-pNA (Cambridge Research Bio-Chemicals) solution (containing 1% DMSO), concentration of which was adjusted to 0.1mM with said buffer, was added thereto, then further reacted for 30 minutes at 30°C, and added 40 μl of acetic acid thereto and measured absorbance at 405nm. An inhibition against an activity of human neutrophil elastase in the samples treated with the oxidizer above were indicated as an inhibitory rate (%) calculated through the following formula.

$$\mathrm{Inhibitory\ Rate\ (\%)} = \frac{A - B}{A} \times 100$$

A : Absorbance obtained from sample not contained inhibitor therein.
B : Absorbance obtained from sample contained inhibitor therein.

Inhibitory rate (%) on α 1-AT, UTI and altered HI-8 of the present invention, against human neutrophil elastase, prepared respectively through the foregoing procedures and treated with the oxidizer were listed in the following Table 3.

Table 3

| Inhibitior (Altered Peptide) | Amount of Inhibitior (p mole) | Inhibitory Rate against Human Neutrophil Elastase (%) | |
|---|---|---|---|
| | | Concentration of chloro-succinate-imide (mM) | |
| | | 0 | 2.5 |
| α 1-AT | 10 | 100 | 6 |
| UTI | 20 | 72 | 5 |
| I15G3 | 5 | 84 | 74 |
| L15G3 | 5 | 84 | 74 |
| V15G3 | 5 | 93 | 87 |
| V15G3E11 | 5 | 97 | 92 |
| V15G3Q46 | 5 | 97 | 97 |
| V15G3E11Q46 | 5 | 95 | 94 |
| I15G3E11 | 5 | 87 | 87 |
| I15G3Q46 | 5 | 94 | 94 |
| I15G3E11Q46 | 5 | 92 | 94 |
| I15G3F18 | 5 | 92 | 90 |

It have been understood that an inhibitory activity of recombinant altered peptide of the present invention against human neutrophil elastase were sufficiently remained under a condition wherein α 1-AT and UTI are oxidized and inactivated completely.

(Industrial Applicability)

As stated above, novel peptides which inhibit elastase would be useful as a drug for prevent ion and cure of diseases by elastase, such as pulmonary emphysema, because it will be expected to obtain a strong inhibitory activity against human neutrophil elastase, and, in comparison with the native elastase inhibitor and chemically synthesized low molecular elastase inhibitor, a strong resistance against oxidization, low immunogenicity, and less toxicity to human.

S E Q U E N C E   L I S T I N G


INFORMATION FOR SEQ ID NO:1:

LENGTH:66

TYPE: Amino Acid

TOPOLOGY: Unknown

MOLECULAR TYPE: Protein

FEATURE

  NAME/KEY: mutation

  LOCATION: 3

  DETERMINING METHOD: E

  OTHER INFORMATION: Ala or Gly


  NAME/KEY: mutation

  LOCATION: 11

  DETERMINING METHOD: E

  OTHER INFORMATION: Arg or Glu


  NAME/KEY: mutation

  LOCATION: 15

  DETERMINING METHOD: E

  OTHER INFORMATION: Arg, Val, Ile or Leu


  NAME/KEY: mutation

  LOCATION: 18

  DETERMINING METHOD: E

  OTHER INFORMATION: Ile or Phe

NAME/KEY : mutation

LOCATION : 46

DETERMINING METHOD : E

OTHER INFORMATION: Tyr or Gln

SEQUENCE DESCRIPTION: SEQ ID NO:1:

Thr Val Xaa Ala Cys Asn Leu Pro Val Ile Xaa Gly Pro Cys Xaa Ala
1               5                   10                  15

Phe Xaa Gln Leu Trp Ala Phe Asp Ala Val Lys Gly Lys Cys Val Leu
            20                  25                  30

Phe Pro Tyr Gly Gly Cys Gln Gly Asn Gly Asn Lys Phe Xaa Ser Glu
            35                  40                  45

Lys Glu Cys Arg Glu Tyr Cys Gly Val Pro Gly Asp Glu Asp Glu Glu
        50                  55                  60

Leu Leu
65


INFORMATION FOR SEQ ID NO:2:

LENGTH:259

TYPE: Nucleic Acid

STRANDEDNESS: Double

TOPOLOGY: Linear

MOLECULAR TYPE: Other Nucleic Acid, Synthesized DNA

FEATURE

NAME/KEY : CDS

LOCATION : 1..201

DETERMINING METHOD : E

NAME/KEY : mat peptide

LOCATION : 1..198

DETERMINING METHOD : E

NAME/KEY : terminator

LOCATION : 210..242

DETERMINING METHOD : S


NAME/KEY : inhibitory-site

LOCATION : 43..45

DETERMINING METHOD : S

SEQUENCE DESCRIPTION: SEQ ID NO:2:

```
ACG GTT GCT GCT TGC AAC CTG CCG GTT ATC CGT GGT CCG TGC CGT GCT          48
Thr Val Ala Ala Cys Asn Leu Pro Val Ile Arg Gly Pro Cys Arg Ala
 1               5                   10                  15

TTC ATC CAG CTG TGG GCT TTC GAC GCT GTT AAA GGT AAA TGC GTT CTG          96
Phe Ile Gln Leu Trp Ala Phe Asp Ala Val Lys Gly Lys Cys Val Leu
            20                  25                  30

TTC CCG TAT GGT GGT TGC CAG GGT AAC GGT AAC AAA TTC TAT TCT GAA          144
Phe Pro Tyr Gly Gly Cys Gln Gly Asn Gly Asn Lys Phe Tyr Ser Glu
            35                  40                  45

AAA GAA TGC CGT GAA TAT TGC GGT GTT CCG GGT GAC GAA GAC GAA GAA          192
Lys Glu Cys Arg Glu Tyr Cys Gly Val Pro Gly Asp Glu Asp Glu Glu
            50                  55                  60

CTG CTG TGATGATCTA GAGCCCAGCC CGCCTAATGA GCGGGCTTTT TTTTGAACAA          248
Leu Leu
    65

AAGGCGGAATT                                                              259
```

INFORMATION FOR SEQ ID NO:3:

LENGTH:51

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:3:

GGTTGCTGCT TGCAACCTGC CGGTTATCCG TGGTCCGTGC CGTGCTTTCA T          51


INFORMATION FOR SEQ ID NO:4:

LENGTH:50

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:4:

CCAGCTGTGG GCTTTCGACG CTGTTAAAGG TAAATGCGTT CTGTTCCCGT          50


INFORMATION FOR SEQ ID NO:5:

LENGTH:52

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:5:

ATGGTGGTTG CCAGGGTAAC GGTAACAAAT TCTATTCTGA AAAAGAATGC CG          52

INFORMATION FOR SEQ ID NO:6:

LENGTH:50

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:6:

TGAATATTGC GGTGTTCCGG GTGACGAAGA CGAAGAACTG CTGTGATGAT          50


INFORMATION FOR SEQ ID NO:7:

LENGTH:50

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:7:

CTAGAGCCCA GCCCGCCTAA TGAGCGGGCT TTTTTTTGAA CAAAAGGCGG          50


INFORMATION FOR SEQ ID NO:8:

LENGTH:63

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:8:

ACAGCTGGAT GAAAGCACGG CACGGACCAC GGATAACCGG CAGGTTGCAA GCAGCAACCG          60

TAC          63

INFORMATION FOR SEQ ID NO:9:

LENGTH:51

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:9:

AACCACCATA CGGGAACAGA ACGCATTTAC CTTTAACAGC GTCGAAAGCC C          51


INFORMATION FOR SEQ ID NO:10:

LENGTH:51

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:10:

AATATTCACG GCATTCTTTT TCAGAATAGA ATTTGTTACC GTTACCCTGG C          51


INFORMATION FOR SEQ ID NO:11:

LENGTH:52

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:11:

TGGGCTCTAG ATCATCACAG CAGTTCTTCG TCTTCGTCAC CCGGAACACC GC          52

INFORMATION FOR SEQ ID NO:12:

LENGTH:44

TYPE: Nucleic Acid

STRANDEDNESS: Single

TOPOLOGY: Linear

MOLECULAR TYPE: Other Nucleic Acid, Synthesized DNA

SEQUENCE DESCRIPTION: SEQ ID NO:12:

AATTCCGCCT TTTGTTCAAA AAAAAGCCCG CTCATTAGGC GGGC                    44


INFORMATION FOR SEQ ID NO:13:

LENGTH:64

TYPE: Nucleic Acid

STRANDEDNESS: Single

TOPOLOGY: Linear

MOLECULAR TYPE: Other Nucleic Acid, Synthesized DNA

FEATURE

   NAME/KEY : sig peptide

   LOCATION : 2..64

   DETERMINING METHOD : S

   OTHER INFORMATION : Coding Strand of Synthesized DNA which codes Omp A

                          Signal Peptide.   BspHI Adhesive-End and Blunt-End

                          is produced by annealing it with Sequence of SEQ.

                          ID. NO.14.

SEQUENCE DESCRIPTION: SEQ ID NO:13:

C ATG AAA AAA ACC GCT ATC GCT ATC GCT GTT GCT CTG GCT GGT TTT          46
  Met Lys Lys Thr Ala Ile Ala Ile Ala Val Ala Leu Ala Gly Phe
   1            5               10             15

```
GCT ACC GTT GCT CAG GCC                                              64

Ala Thr Val Ala Gln Ala

              20


INFORMATION FOR SEQ ID NO:14:

LENGTH:60

TYPE: Nucleic Acid

STRANDEDNESS: Single

TOPOLOGY: Linear

MOLECULAR TYPE: Other Nucleic Acid, Synthesized DNA

FEATURE

   NAME/KEY : sig peptide

   LOCATION : 1..60

   DETERMINING METHOD : S

   OTHER INFORMATION : Complementary Strand is formed with Sequence of

                       SEQ. ID. NO. 13.

SEQUENCE DESCRIPTION: SEQ ID NO:14:

GGCCTGAGCA ACGGTAGCAA AACCAGCCAG AGCAACAGCG ATAGCGATAG CGGTTTTTTT    60


INFORMATION FOR SEQ ID NO:15:

LENGTH:18

TYPE: Amino Acid

TOPOLOGY: Linear

MOLECULAR TYPE: Peptide

SEQUENCE DESCRIPTION: SEQ ID NO:15:

Ser Glu Lys Glu Cys Arg Glu Tyr Cys Gly Val Pro Gly Asp Glu Asp

 1               5                 10                15

Glu Glu
```

INFORMATION FOR SEQ ID NO:16:

LENGTH:18

TYPE: Nucleic Acid

STRANDEDNESS: Single

TOPOLOGY: Linear

MOLECULAR TYPE: Other Nucleic Acid, Synthesized DNA

FEATURE

   NAME/KEY : mutation

   LOCATION : 7..9

   DETERMINING METHOD : S

   OTHER INFORMATION : Third Amino Acid of Recombinant HI-8 is converted

                       into Glycine.

SEQUENCE DESCRIPTION: SEQ ID NO:16:

ACGGTTGGTG CTTGCAAC                                18


INFORMATION FOR SEQ ID NO:17:

LENGTH:21

TYPE: Nucleic Acid

STRANDEDNESS: Single

TOPOLOGY: Linear

MOLECULAR TYPE: Other Nucleic Acid, Synthesized DNA

FEATURE

   NAME/KEY : mutation

   LOCATION : 10..12

   DETERMINING METHOD : S

   OTHER INFORMATION : 15th Amino Acid of Recombinant HI-8 is converted

                       into Isoleucine.

SEQUENCE DESCRIPTION: SEQ ID NO:17:

GGTCCGTGCA TTGCTTTCAT C                                        21

INFORMATION FOR SEQ ID NO:18:

LENGTH:21

TYPE: Nucleic Acid

STRANDEDNESS: Single

TOPOLOGY: Linear

MOLECULAR TYPE: Other Nucleic Acid, Synthesized DNA

FEATURE

   NAME/KEY : mutation

   LOCATION : 10..12

   DETERMINING METHOD : S

   OTHER INFORMATION  : 15th Amino Acid of Recombinant HI-8 is converted

                        into Leucine.

SEQUENCE DESCRIPTION: SEQ ID NO:18:

GGTCCGTGCC TGGCTTTCAT C                                        21

INFORMATION FOR SEQ ID NO:19:

LENGTH:21

TYPE: Nucleic Acid

STRANDEDNESS: Single

TOPOLOGY: Linear

MOLECULAR TYPE: Other Nucleic Acid, Synthesized DNA

FEATURE

   NAME/KEY : mutation

   LOCATION : 10..12

   DETERMINING METHOD : S

OTHER INFORMATION : 15th Amino Acid of Recombinant HI-8 is converted into Valine.

SEQUENCE DESCRIPTION: SEQ ID NO:19:

GGTCCGTGCG TTGCTTTCAT C                                                      21


INFORMATION FOR SEQ ID NO:20:

LENGTH:21

TYPE: Nucleic Acid

STRANDEDNESS: Single

TOPOLOGY: Linear

MOLECULAR TYPE: Other Nucleic Acid, Synthesized DNA

FEATURE

  NAME/KEY : mutation

  LOCATION : 10..12

  DETERMINING METHOD : S

  OTHER INFORMATION : 11th Amino Acid of Recombinant HI-8 is converted into Glutamic Acid.

SEQUENCE DESCRIPTION: SEQ ID NO:20:

CCGGTTATCG AAGGTCCGTG C                                                      21


INFORMATION FOR SEQ ID NO:21:

LENGTH:36

TYPE: Nucleic Acid

STRANDEDNESS: Single

TOPOLOGY: Linear

MOLECULAR TYPE: Other Nucleic Acid, Synthesized DNA

FEATURE

  NAME/KEY : mutation

LOCATION : 17..19

DETERMINING METHOD : S

OTHER INFORMATION : 46th Amino Acid of Recombinant HI-8 is converted into Glutamine.

SEQUENCE DESCRIPTION: SEQ ID NO:21:

TAACGGTAAC AAATTCCAGT CTGAAAAAGA ATGCCG                                  36


INFORMATION FOR SEQ ID NO:22:

LENGTH:24

TYPE: Nucleic Acid

STRANDEDNESS: Single

TOPOLOGY: Linear

MOLECULAR TYPE: Other Nucleic Acid, Synthesized DNA

FEATURE

  NAME/KEY : mutation

  LOCATION : 13..15

  DETERMINING METHOD : S

  OTHER INFORMATION : 18th Amino Acid of Recombinant HI-8, wherein 15th Amino Acid was already altered to Isoleucine, is further converted into Phenylalanine.

SEQUENCE DESCRIPTION: SEQ ID NO:22:

TGCATTGCTT TCTTCCAGCT GTGG                                               24


**Claims**

1.  A peptide having an elastase inhibitory activity comprising an amino acid sequence of;

```
Thr-Val-Gly-Ala-Cys-Asn-Leu-Pro-Val-Ile-X11-Gly-Pro-Cys-

X15-Ala-Phe-X18-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-X46-Ser-Glu-Lys-Glu-Cys-Arg-Glu-Tyr-Cys-Gly-

Val-Pro-Gly-Asp-Glu-Asp-Glu-Glu-Leu-Leu  ﹑
```

wherein
   X11 is Arg or Glu;
   X15 is Ile, Leu or Val;
   X18 is Ile or Phe; and
   X46 is Tyr or Gln.

2.   The peptide according to claim 1, wherein said amino acid sequence is;

```
Thr-Val-Gly-Ala-Cys-Asn-Leu-Pro-Val-Ile-Arg-Gly-Pro-Cys-

Ile-Ala-Phe-Ile-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Tyr-Ser-Glu-Lys-Glu-Cys-Arg-Glu-Tyr-Cys-Gly-

Val-Pro-Gly-Asp-Glu-Asp-Glu-Glu-Leu-Leu.
```

3.   The peptide according to claim 1, wherein said amino acid sequence is;

```
Thr-Val-Gly-Ala-Cys-Asn-Leu-Pro-Val-Ile-Arg-Gly-Pro-Cys-

Leu-Ala-Phe-Ile-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Tyr-Ser-Glu-Lys-Glu-Cys-Arg-Glu-Tyr-Cys-Gly-

Val-Pro-Gly-Asp-Glu-Asp-Glu-Glu-Leu-Leu.
```

4.   The peptide according to claim 1, wherein said amino acid sequence is;

```
Thr-Val-Gly-Ala-Cys-Asn-Leu-Pro-Val-Ile-Arg-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Tyr-Ser-Glu-Lys-Glu-Cys-Arg-Glu-Tyr-Cys-Gly-
Val-Pro-Gly-Asp-Glu-Asp-Glu-Glu-Leu-Leu.
```

**5.** The peptide according to claim 1, wherein said amino acid sequence is;

```
Thr-Val-Gly-Ala-Cys-Asn-Leu-Pro-Val-Ile-Glu-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Tyr-Ser-Glu-Lys-Glu-Cys-Arg-Glu-Tyr-Cys-Gly-
Val-Pro-Gly-Asp-Glu-Asp-Glu-Glu-Leu-Leu.
```

**6.** The peptide according to claim 1, wherein said amino acid sequence is;

```
Thr-Val-Gly-Ala-Cys-Asn-Leu-Pro-Val-Ile-Arg-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

**7.** The peptide according to claim 1, wherein said amino acid sequence is;

```
Thr-Val-Gly-Ala-Cys-Asn-Leu-Pro-Val-Ile-Glu-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

26

8. The peptide according to claim 1, wherein said amino acid sequence is;

Thr-Val-Gly-Ala-Cys-Asn-Leu-Pro-Val-Ile-Glu-Gly-Pro-Cys-

Ile-Ala-Phe-Ile-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Tyr-Ser-Glu-Lys-Glu-Cys-Arg-Glu-Tyr-Cys-Gly-

Val-Pro-Gly-Asp-Glu-Asp-Glu-Glu-Leu-Leu.

9. The peptide according to claim 1, wherein said amino acid sequence is;

Thr-Val-Gly-Ala-Cys-Asn-Leu-Pro-Val-Ile-Arg-Gly-Pro-Cys-

Ile-Ala-Phe-Ile-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Gln-Ser-Glu-Lys-Glu-Cys-Arg-Glu-Tyr-Cys-Gly-

Val-Pro-Gly-Asp-Glu-Asp-Glu-Glu-Leu-Leu.

10. The peptide according to claim 1, wherein said amino acid sequence is;

Thr-Val-Gly-Ala-Cys-Asn-Leu-Pro-Val-Ile-Glu-Gly-Pro-Cys-

Ile-Ala-Phe-Ile-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Gln-Ser-Glu-Lys-Glu-Cys-Arg-Glu-Tyr-Cys-Gly-

Val-Pro-Gly-Asp-Glu-Asp-Glu-Glu-Leu-Leu.

11. The peptide according to claim 1, wherein said amino acid sequence is;

Thr-Val-Gly-Ala-Cys-Asn-Leu-Pro-Val-Ile-Arg-Gly-Pro-Cys-

Ile-Ala-Phe-Phe-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-Tyr-Ser-Glu-Lys-Glu-Cys-Arg-Glu-Tyr-Cys-Gly-

Val-Pro-Gly-Asp-Glu-Asp-Glu-Glu-Leu-Leu.

**12.** A gene which codes an amino acid sequence of:

Thr-Val-Gly-Ala-Cys-Asn-Leu-Pro-Val-Ile-X11-Gly-Pro-Cys-

X15-Ala-Phe-X18-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-X46-Ser-Glu-Lys-Glu-Cys-Arg-Glu-Tyr-Cys-Gly-

Val-Pro-Gly-Asp-Glu-Asp-Glu-Glu-Leu-Leu,

wherein
X11 is Arg or Glu;
X15 is Ile, Leu or Val;
X18 is Ile or Phe; and
X46 is Tyr or Gln.

**13.** A plasmid comprising a gene which codes an amino acid sequence of:

Thr-Val-Gly-Ala-Cys-Asn-Leu-Pro-Val-Ile-X11-Gly-Pro-Cys-

X15-Ala-Phe-X18-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-X46-Ser-Glu-Lys-Glu-Cys-Arg-Glu-Tyr-Cys-Gly-

Val-Pro-Gly-Asp-Glu-Asp-Glu-Glu-Leu-Leu,

wherein
X11 is Arg or Glu;
X15 is Ile, Leu or Val;
X18 is Ile or Phe; and
X46 is Tyr or Gln.

**14.** A host microorganism having a plasmid comprising a gene which codes an amino acid sequence of:

Thr-Val-Gly-Ala-Cys-Asn-Leu-Pro-Val-Ile-X11-Gly-Pro-Cys-

X15-Ala-Phe-X18-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-X46-Ser-Glu-Lys-Glu-Cys-Arg-Glu-Tyr-Cys-Gly-

Val-Pro-Gly-Asp-Glu-Asp-Glu-Glu-Leu-Leu,

wherein
X11 is Arg or Glu;
X15 is Ile, Leu or Val;
X18 is Ile or Phe; and
X46 is Tyr or Gln.

15. The host microorganism according to claim 14, wherein said amino acid sequence is;

```
Thr-Val-Gly-Ala-Cys-Asn-Leu-Pro-Val-Ile-Arg-Gly-Pro-Cys-
Ile-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Tyr-Ser-Glu-Lys-Glu-Cys-Arg-Glu-Tyr-Cys-Gly-
Val-Pro-Gly-Asp-Glu-Asp-Glu-Glu-Leu-Leu.
```

16. The host microorganism according to claim 15, wherein said host microorganism is <u>Escherichia coli</u> JM109-pCD17I15G3 (FERM BP-4556).

17. The host microorganism according to claim 14, wherein said amino acid sequence is;

```
Thr-Val-Gly-Ala-Cys-Asn-Leu-Pro-Val-Ile-Arg-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

18. The host microorganism according to claim 17, wherein said host microorganism is <u>Escherichia coli</u> JM109-pCD17V15G3Q46 (FERM BP-4557).

19. The host microorganism according to claim 14, wherein said amino acid sequence is;

```
Thr-Val-Gly-Ala-Cys-Asn-Leu-Pro-Val-Ile-Glu-Gly-Pro-Cys-
Val-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

20. The host microorganism according to claim 19, wherein said host microorganism is <u>Escherichia coli</u> JM109-pCD17V15G3E11Q46 (FERM BP-4560).

21. The host microorganism according to claim 14, wherein said amino acid sequence is;

```
Thr-Val-Gly-Ala-Cys-Asn-Leu-Pro-Val-Ile-Arg-Gly-Pro-Cys-
Ile-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

**22.** The host microorganism according to claim 21, wherein said host microorganism is <u>Escherichia coli</u> JM109-pCD17I15G3Q46 (FERM BP-4558).

**23.** The host microorganism according to claim 14, wherein said amino acid sequence is;

```
Thr-Val-Gly-Ala-Cys-Asn-Leu-Pro-Val-Ile-Glu-Gly-Pro-Cys-
Ile-Ala-Phe-Ile-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-Gln-Ser-Glu-Lys-Glu-Cys-Arg-Glu-Tyr-Cys-Gly-
Val-Pro-Gly-Asp-Glu-Asp-Glu-Glu-Leu-Leu.
```

**24.** The host microorganism according to claim 23, wherein said host microorganism is <u>Escherichia coli</u> JM109-pCD17I15G3E11Q46 (FERM BP-4559).

**25.** A peptide product having an elastase inhibitory activity produced by a host microorganism having a plasmid comprising a gene which codes an amino acid sequence of:

```
Thr-Val-Gly-Ala-Cys-Asn-Leu-Pro-Val-Ile-X11-Gly-Pro-Cys-
X15-Ala-Phe-X18-Gln-Leu-Trp-Ala-Phe-Asp-Ala-Val-Lys-Gly-
Lys-Cys-Val-Leu-Phe-Pro-Tyr-Gly-Gly-Cys-Gln-Gly-Asn-Gly-
Asn-Lys-Phe-X46-Ser-Glu-Lys-Glu-Cys-Arg-Glu-Tyr-Cys-Gly-
Val-Pro-Gly-Asp-Glu-Asp-Glu-Glu-Leu-Leu.
```

wherein
    X11 is Arg or Glu;
    X15 is Ile, Leu or Val;
    X18 is Ile or Phe; and
    X46 is Tyr or Gln.

**26.** A method for producing a peptide product having an elastase inhibitory activity produced by a host microorganism having a plasmid comprising a gene which codes an amino acid sequence of:

```
Thr-Val-Gly-Ala-Cys-Asn-Leu-Pro-Val-Ile-X11-Gly-Pro-Cys-

X15-Ala-Phe-X18-Gln-Leu-Trp-Ala-Phe-Asp-Ala-Val-Lys-Gly-

Lys-Cys-Val-Leu-Phe-Pro-Tyr-Gly-Gly-Cys-Gln-Gly-Asn-Gly-

Asn-Lys-Phe-X46-Ser-Glu-Lys-Glu-Cys-Arg-Glu-Tyr-Cys-Gly-

Val-Pro-Gly-Asp-Glu-Asp-Glu-Glu-Leu-Leu,
```

wherein
  X11 is Arg or Glu;
  X15 is Ile, Leu or Val;
  X18 is Ile or Phe; and
  X46 is Tyr or Gln.
comprising the steps of:
  (a) culturing said host microorganism on growth medium;
  (b) harvesting the bacterial cells cultured in said step (a) as a precipitate by a centrifugation; and
  (c) extracting and collecting peptide products having elastase inhibitory activity from the bacterial cells collected in said step (b).

EP 0 643 075 A1

5'- GGTTGCTGCTTGCAACCTGCCGGTTATCCGTGGTCCGTGCCGTGCTTTCATCCAGCTGTGGGCTTTCGACGCTGTTAAAGGTAAATGCGTTC
3'- CATGCCAACGACGAACGTTGGACGGCCAATAGGCACCAGGCACGGCACGAAAGTAGGTCGACACCCGAAAGCTGCGACAATTTCCATTTACGCAAG

(1)    (2)    (6)    (7)

TGTTCCCGTATGGTGGTTGCCAGGGTAACGGTAACAAAATTCTATTCTGAAAAAGAATGCCGTGAATATTGCGGTGTTCCGGGTGACGAAGACGAAG
ACAAGGGCATACCACCAACGGTCCCATTGCCATTGTTTAAGATAAGACTTTTTCTTACGGCACTTATAACGCCACAAGGCCCACTGCTTCTGCTTC

(3)    (4)    (8)    (9)

AACTGCTGTGATGATCTAGAGCCCAGCCCGCCTAATGAGCGGGCTTTTTTTTGAACAAAAGGCGG -3'
TTGACGACACTACTAGATCTCGGGTCGGGCGGATTACTCGCCCGAAAAAAAACTTGTTTTCCGCCTAA -5'

(5)    (10)

Fig.1

Fig.2

| | International application No. |
|---|---|
| | PCT/JP94/00284 |

## A. CLASSIFICATION OF SUBJECT MATTER

Int. Cl$^5$ C07K7/10, C12N15/15, C12N1/21, C12P21/02

According to International Patent Classification (IPC) or to both national classification and IPC

## B. FIELDS SEARCHED

Minimum documentation searched (classification system followed by classification symbols)

Int. Cl$^5$ C07K7/10, C12N15/15, C12N1/21, C12P21/02

Documentation searched other than minimum documentation to the extent that such documents are included in the fields searched

Electronic data base consulted during the international search (name of data base and, where practicable, search terms used)

CAS ONLINE

## C. DOCUMENTS CONSIDERED TO BE RELEVANT

| Category* | Citation of document, with indication, where appropriate, of the relevant passages | Relevant to claim No. |
|---|---|---|
| X | CHEMICAL ABSTRACTS, Vol. 105, No. 3, the abstract No. 17700n; H. Ohnishi et. al.: "Pharmacological activities of a" trypsin inhibitor, urinastatin (Mivaclid) & Oyo Yakuri 1986, 31(3), P. 663-75 | 1-26 |

☐ Further documents are listed in the continuation of Box C.       ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| May 12, 1994 (12. 05. 94) | May 31, 1994 (31. 05. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)